# NOUVEAU FASCICULE DE BREVET EUROPEEN

(11) **EP 2 379 693 B2**
(45) Date de publication et mention de la décision concernant l'opposition: **16.10.2024**
(45) Mention de la délivrance du brevet: 21.08.2019
(21) Numéro de dépôt: 10707570.7
(22) Date de dépôt: 22.01.2010
(51) Int. Cl.: C12M 1/00, C08J 7/04

(54) **UTILISATION D'UNE COMPOSITION TRANSPARENTE POUR PHOTOBIOREACTEURS**
VERWENDUNG EINER TRANSPARENTEN ZUSAMMENSETZUNG FÜR PHOTOBIOREAKTOREN
USE OF A TRANSPARENT COMPOSITION FOR PHOTOBIOREACTORS

(30) Priorité: 22.01.2009 FR 0950374; 17.07.2009 FR 0954969
(43) Date de publication de la demande: 26.10.2011
(73) Titulaire: Arkema France, 92700 Colombes (FR)
(72) Inventeur: DELPRAT, Patrick, F-64230 Lescar (FR); BERGE, Jérôme, F-64360 Parbayse (FR); BOUTILLIER, Jean-Marc, F-64230 SAUVAGNON (FR)
(74) Mandataire: SSM Sandmair
(86) Numéro de dépôt international: PCT/FR2010/050095
(87) Numéro de publication internationale: WO 2010/084289

(56) Documents cités:
- EP-A2- 0 239 272
- WO-A1-2009/000566
- WO-A2-2007/025145
- WO-A2-2008/115058
- JP-A- H11 291 415
- DATABASE WPI Week 199303, Derwent World Patents Index; AN 1993-023443, XP002549153

## Description

La présente invention concerne les photobioréacteurs et a plus particulièrement pour objet une structure multicouche transparente comprenant au moins une couche d'un polymère méthacrylique pour la culture d'organismes photosensibles comme défini par des revendications.

Un photobioréacteur est un système dans lequel se déroulent, en présence d'énergie lumineuse, des interactions biologiques que l'on cherche à contrôler en maîtrisant les conditions de culture. En son sein, une réaction biochimique de photosynthèse a lieu dans le but de produire de la biomasse végétale à partir de microorganismes photosynthétiques, de lumière, de gaz carbonique et d'un minimum d'éléments minéraux. Lors du mécanisme de photosynthèse, l'essentiel des composants organiques est fabriqué (glucides, lipides et protéines).

Les microalgues, photosynthétiques sous le rayonnement solaire, permettent en aquaculture la production d'une biomasse dont la composition dépend de l'espèce choisie, et qui peut atteindre en valeur énergétique, selon les estimations, vingt à cent fois celle des plantes au sol. On compte quelques milliers de genres de microalgues répartis en espèces dont certaines représentent un large groupe potentiellement fournisseur de composés valorisables comme biocarburants ou dans les domaines de la cosmétique, la pharmacie, voire l'agro-alimentaire.

Certaines espèces sont riches en lipides, il est possible d'en extraire les huiles (triglycérides) donnant plus ou moins directement un biodiesel. Les résidus peuvent aussi être valorisés, par exemple par une fermentation produisant du bioéthanol. Les microalgues riches en lipides deviennent ainsi une solution technique aux problèmes énergétiques liés à l'épuisement des gisements de pétrole et au remplacement des matières premières d'origine fossile par des matières renouvelables. De plus, la production d'algues est accélérée par barbotage de CO₂ issu par exemple d'une industrie polluante comme une centrale électrique thermique à flamme, évitant ainsi le rejet immédiat de ce gaz à effet de serre. Les microalgues constituent également une alternative à l'obtention de biocarburants à partir de cultures végétales (par exemple colza, betteraves, blé) qui nécessitent d'importantes surfaces cultivables. Selon des scientifiques américains, certaines microalgues seraient capables de synthétiser 30 fois plus d'huile à l'hectare que les plantes terrestres utilisées pour la fabrication de biocarburants. Par ailleurs, l'utilisation de cette matière première comme source de biocarburants évite les problèmes de saisonnalité et d'approvisionnement caractéristiques de l'emploi des végétaux terrestres.

Composées essentiellement de protéines, certaines espèces présentent un bon précédent alimentaire car elles sont riches en vitamines, en acides gras polyinsaturés et en oligo-éléments. Elles trouvent des applications dans l'agriculture et l'horticulture par l'utilisation d'extraits d'algues qui assurent un rôle non seulement de fertilisant mais aussi d'accélérateur et protecteur de cultures.

D'autres espèces représentent un potentiel productif applicable dans les domaines de la santé, pour la synthèse de médicaments ou de biocosmétiques à partir des extraits de substances à activités biologique ou thérapeutique, dans l'industrie agro-alimentaire par l'extraction et fabrication de pigments, de colorants naturels ou de gélifiants. Les microalgues peuvent constituer des sources potentielles de molécules difficilement accessibles par synthèse chimique.

Les microalgues peuvent être directement impliquées dans la production d'énergie nouvelle et renouvelable notamment la production de biocombustibles comme l'hydrogène, Certaines espèces peuvent produire de l'hydrogène sous l'action des enzymes présentes du type hydrogénases.

La culture de microalgues offre une alternative intéressante pour le traitement des eaux usées (effluents urbains, industriels ou agricoles). Elle permet un biotraitement tertiaire couplé à la production de biomasse potentiellement valorisable. Comme agent épurateur, les microalgues jouent divers rôles tels que l'élimination simultanée des sels nutritifs (NH⁴⁺, NO³⁻, PO₄³⁻), l'épuration des effluents secondaires par production d'oxygène qui est utilisé par les bactéries pour dégrader des composés organiques résiduels, une action bactéricide réduisant la survie des germes pathogènes contenus dans les effluents secondaires. Cette technologie a l'avantage de reposer sur les principes des écosystèmes naturels et est donc sans danger pour l'environnement.

La croissance des microalgues peut être réalisée à la surface de bassins solaires ouverts ou sous des serres, ce qui constitue des photobioréacteurs d'investissement limité. Cependant, cette technologie est limitée à l'utilisation d'espèces robustes, pouvant résister aux variations de température ou d'ensoleillement ou à des virus. La production de biodiesel à partir de la biomasse ainsi obtenue, ne s'est pas avérée économiquement compétitive par rapport aux produits pétroliers.

Pour une production plus stable ou plus intensive, on utilise des photobioréacteurs solaires fermés, à parois transparentes. Ces appareils sont généralement de géométrie plane (photobioréacteurs plats) ou de géométrie cylindrique (photobioréacteurs tubulaires). On peut également utiliser des photobioréacteurs avec une géométrie plus particulière telle que des structures de type profilé creux en forme de « H » ou de « I » par exemple.

Un photobioréacteur plat se compose de deux panneaux parallèles transparents, de surfaces variables et entre lesquels réside une mince couche de culture d'une profondeur de quelques centimètres.

Un photobioréacteur tubulaire se compose d'un ou plusieurs tubes transparents, de diamètres et de longueurs variables, de configurations diverses et au sein desquels peut circuler la culture. Les variantes de configuration sont multiples :
- un tube large et vertical formant une colonne,
- deux tubes de diamètres différents agencés l'un dans l'autre formant une chambre annulaire,
- un tube placé au sol et de diamètre modéré mais de longueur importante, agencé sous forme de serpentin,
- un tube de petit diamètre et de longueur importante enroulé hélicoïdalement autour d'une tour,
- plusieurs tubes de petit diamètre agencés parallèlement et à la verticale.

La particularité d'un photobioréacteur tient, en plus des besoins habituels communs à tous les bioréacteurs, à la nécessité de fournir une énergie photonique aux microorganismes à cultiver, en particulier les microalgues, cet apport étant incontournable pour la réalisation de la photosynthèse. D'un point de vue technologique, l'enceinte du système doit donc être transparente et être conçue de façon à fournir une intensité lumineuse suffisante pour les microalgues.

Pour répondre à cette exigence, les photobioréacteurs sont généralement fabriqués à partir d'un matériau présentant une grande transparence dans le domaine du visible, tel que le verre ou le polycarbonate (PC).

L'utilisation du verre qui en outre doit être de haute pureté, présente cependant de nombreux inconvénients : c'est un matériau lourd, cher, rigide qui se casse facilement et qui est difficile à usiner. Le verre présente de plus une diffusion de lumière moins bonne que les polymères méthacryliques tel que le PMMA. Sa mise en oeuvre limite le choix de la géométrie du réacteur : les agencements sous forme de boucles ou de serpentins sont difficilement réalisables, les tubes connectés les uns aux autres par des tubulures ou des raccords sont sources de fuite ; afin de maintenir un accès correct de la lumière, les réacteurs doivent être nettoyés très souvent en raison de la formation d'un biofilm sur leurs parois. L'optimisation entre une grande longueur de tube, une surface réduite au sol et l'accessibilité de la lumière au milieu de culture s'avère difficile.

Le polycarbonate est un produit qui résiste peu aux UV dans le temps, et dont la transmission de la lumière est inférieure à celle du PMMA. Par ailleurs, pour les photobioréacteurs qui nécessitent des parois de forte épaisseur, le PC devient très fragile. Enfin, le PC présente une faible résistance chimique envers les produits de lavage tels que l'acide chlorhydrique, l'eau de Javel et l'ozone.

La géométrie du réacteur doit autant que possible privilégier un rapport élevé de la surface éclairée sur le volume de culture, tout en limitant l'encombrement et en restant en adéquation avec les objectifs de concentration en biomasse souhaitée et des besoins physiologiques des microorganismes cultivés. Un compromis hydrodynamique/ régulation est à rechercher également pour assurer un mélange suffisant et un contrôle des paramètres tels que consommation de CO₂, dégagement d'oxygène, température.

L'accès à la lumière doit être optimisé dans tous les cas, de façon à ce que la lumière réellement disponible soit importante et homogène au sein de la culture. Différentes variantes sont utilisées, par exemple incliner les réacteurs plats pour améliorer l'utilisation de l'irradiance solaire, placer les réacteurs sur des surfaces réfléchissantes afin d'accroître par réflexion l'incidence du rayonnement, disposer des sources lumineuses artificielles dans des tubes au coeur du milieu de culture, etc.

Les photobioréacteurs donnent lieu à de nombreuses publications, par exemple les documents suivants décrivent différentes configurations pour mettre en oeuvre des réactions de photosynthèse à partir de microorganismes : EP 112 556 ; EP 239 272 ; WO 99/20736 ; WO 00/12673 ; WO 00/23562 ; FR 2 907 311 ; WO 07/025145 ; WO 08/040828 ; US 2005/064577.

Le document WO 2008/115058 décrit une installation pour la culture d'algues utilisant en outre des polymères méthacryliques (PMMA), sans des détails sur les caractéristiques de ledit PMMA.

Concernant la nature des matériaux transparents utilisés pour élaborer les zones de culture, tubes ou chambres de réaction pour la photosynthèse de microorganismes, l'état de la technique propose différents matériaux parmi les matériaux plastiques tels que le polyéthylène (PE), le polycarbonate (PC), le poly acrylate de méthyle (PMA), les polymères méthacryliques tel que le poly méthacrylate de méthyle (PMMA), l'acétate-butyrate de cellulose (CAB), le polychlorure de vinyle (PVC), le polyéthylène téréphtalate (PET), le polyéthylène téréphtalate modfié glycol (PETG), ou des matériaux non plastiques tel que le verre.

La présente invention a pour objet de proposer un matériau transparent susceptible de remplacer le verre ou le polycarbonate pour la culture d'organismes photosensibles dans des photobioréacteurs, qui permet d'éviter les problèmes techniques et inconvénients précités rencontrés avec l'utilisation actuelle du verre ou du polycarbonate, et permet d'améliorer son efficacité et sa durée de vie.

L'efficacité du réacteur sera d'autant plus élevée que le matériau qui le constitue laissera passer la lumière et que cette diffusion de lumière reste élevée dans le temps. Par ailleurs, lors des opérations de nettoyage, il faut éviter de rayer la surface du réacteur et s'assurer que le réacteur ne va se fissurer ou casser par attaque chimique du matériau par les produits de lavage.

La présente invention a pour objet de fournir un matériau thermoplastique léger et facilement transportable, transparent dans le domaine du visible (entre 400 et 800 nm), résistant au cours du temps, notamment sous l'effet d'une exposition prolongée au soleil ou à l'humidité, présentant une tenue mécanique élevée, une bonne résistance chimique aux produits de lavage, transformable en différentes formes, notamment flexibles et raccordables.

Il a été trouvé de façon surprenante qu'un polymère méthacrylique de type PMMA permet d'allier les meilleures performances par rapport aux matériaux plastiques PC, PE, PVC et PET, et le verre, sur les aspects précités, en particulier sur les différents aspects suivants : transparence, durabilité en exposition extérieure, résistance à la rayure, résistance chimique, poids et coût.

La présente invention a pour but de fournir des plaques, profilés ou tubes transparents destinés à la construction d'installations de géométries et configurations diverses pour la culture d'organismes photosensibles.

Plus précisément, la présente invention a pour objet l'utilisation d'une composition transparente à base d'au moins un polymère méthacrylique pour la construction d'installations destinées à la culture d'organismes photosensibles, tels que microorganismes, microalgues, bactéries photosynthétiques ou planctons.

L'invention est caractérisée en ce que le polymère méthacrylique, est le PMMA, homopolymère du méthacrylate de méthyle MAM - ou un copolymère du méthacrylate de méthyle (MAM), comprenant en poids au moins 50% de MAM. Le copolymère est obtenu à partir de MAM et d'au moins un comonomère copolymérisable avec le MAM. De préférence, le copolymère comprend en poids de 70 à 99,9%, avantageusement de 90 à 99,9%, de préférence de 95 à 99,9% de MAM pour respectivement de 0,1 à 30%, avantageusement de 0,1 à 10%, de préférence de 0.1 à 5% de comonomère.

De préférence, le comonomère copolymérisable avec le MAM est un monomère (méth)acrylique ou un monomère vinylaromatique tel que par exemple le styrène ou les styrènes substitués.

Le comonomère peut être choisi par exemple dans la liste des :
- monomères acryliques de formule CH₂=CH-C(=O)-O-R₁ où R₁ désigne un atome d'hydrogène, un groupement alkyle en C₁-C₄₀ linéaire, cyclique ou ramifié éventuellement substitué par un atome d'halogène, un groupement hydroxy, alcoxy, cyano, amino ou époxy tels que par exemple l'acide acrylique, l'acrylate de méthyle, d'éthyle, de propyle, de n-butyle, d'isobutyle, de tertiobutyle, de 2-éthylhexyle, de glycidyle, les acrylates d'hydroxyalkyle, l'acrylonitrile ;
- les monomères méthacryliques de formule CH₂=C(CH₃)-C(=O)-O-R₂ où R₂ désigne un atome d'hydrogène, un groupement alkyle en C₂-C₄₀ linéaire, cyclique ou ramifié éventuellement substitué par un atome d'halogène, un groupement hydroxy, alcoxy, cyano, amino ou époxy tels que par exemple l'acide méthacrylique, le méthacrylate de méthyle, d'éthyle, de propyle, de n-butyle, d'isobutyle, de tertiobutyle, de 2-éthylhexyle, de glycidyle, les méthacrylates d'hydroxyalkyle, le méthacrylonitrile ;
- les monomères vinylaromatiques tels que par exemple le styrène, les styrènes substitués, l'alpha-méthylstyrène, le monochlorostyrène, le tertbutyl styrène.

Le comonomère peut être aussi un agent réticulant c'est-à-dire une molécule ou un oligomère présentant au moins deux insaturations éthyléniques, polymérisables avec le MAM par un mécanisme radicalaire. L'agent réticulant peut être difonctionnel. Il peut s'agir par exemple du di(méth)acrylate d'éthylène glycol, d'hexanediol, de tripropylène glycol, de butanediol, de néopéntyl glycol, de diéthylène glycol, de triéthylène glycol, de dipropylène glycol, d'allyle ou de divinyl benzène. L'agent réticulant peut être aussi trifonctionnel. Il peut s'agir par exemple du tri(méth)acrylate de tripropylène glycol, de triméthylol propane, de pentaérythritol. L'agent réticulant peut être aussi tétrafonctionnel, comme par exemple le tétra(méth)acrylate de pentaérythritol ou hexafonctionnel comme l'hexa(méth)acrylate de dipentaérythritol.

De préférence, le comonomère est un (méth)acrylate d'alkyle, notamment l'acrylate de méthyle, d'éthyle, de propyle, de butyle, ou le méthacrylate de butyle.

Le PMMA pourra être avantageusement un copolymère du MAM et de l'acide acrylique et/ou méthacrylique. Ce type de PMMA offre une résistance thermomécanique ainsi qu'une résistance à la rayure améliorée par rapport à un PMMA n'en contenant pas.

Le PMMA présente des caractéristiques de transmission lumineuse supérieures à celles des autres polymères transparents et du verre, comme le montre le tableau ci-après :

| | Transmission, % Norme ASTM D1003 |
|---|---|
| PMMA Standard | 92 |
| PMMA impact | 90-92 |
| Verre | 90 |
| PC | 84-88 |
| SAN | 84-89 |
| ABS Transparent | 80-89 |
| PA | 86 |
| PS | 89 |
| Copolyesters et PET | 82-91 |

Le PMMA présente des propriétés de résistance à la rayure supérieures à celles des autres polymères transparents, comme l'indique le tableau ci-après :

| | Test crayon* Norme ASTM D3363 |
|---|---|
| PMMA Standard | 2H - 4H |
| PMMÀ impact | B - 2H |
| PC | 3B - HB |
| SAN, ABS Transparent, PA, PS, PVC, PET, PA, | < B |
| * Dureté des mines disponibles (du plus mou au plus dur): 7B-6B-5B-4B-3B-2B-B-HB-F-H-2H-3H-4H-5H-6H | |

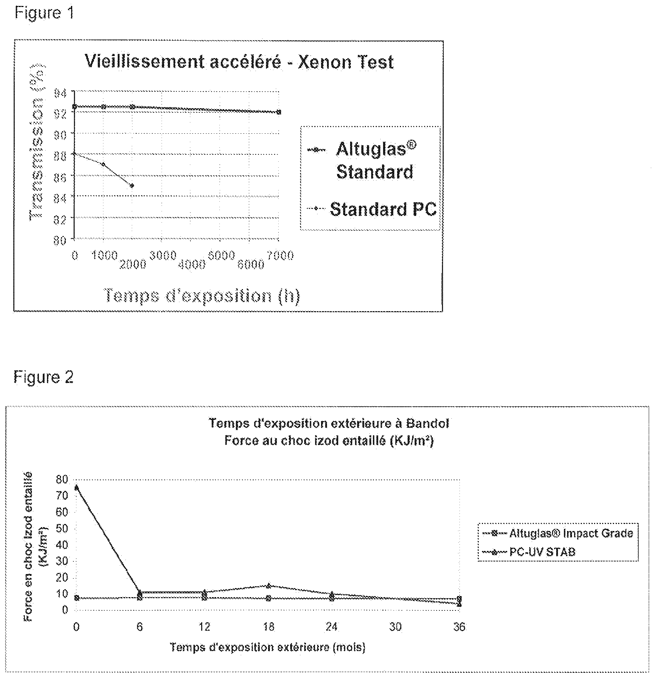

De plus, les figures 1 et 2 illustrent les propriétés supérieures du PMMA (grade standard) par rapport à un polycarbonate PC standard en termes de durabilité en exposition extérieure et résistance aux UV.
La figure 1 représente l'évolution de la transmission lumineuse des 2 matériaux testés en fonction de la durée de vieillissement accéléré dans une enceinte climatique, selon la méthode ISO 4892.
La figure 2 illustre la perte des propriétés mécaniques du PC après 6 mois d'exposition extérieure, selon la méthode NF T51-165.

Le polymère méthacrylique selon l'invention a une masse moléculaire en poids (M_{w}) allant de 90000 g/mol à 170000 g/mol, avantageusement de 110000 à 170000 g/mol, de préférence de 140000 g/mol à 160000 g/mol (étalon équivalent PMMA calculé à base du standard PS). Selon l'invention, la masse moléculaire en poids est déterminée selon la méthode par chromatographie par perméation de gel (GPC, Gel Permeation Chromatography), ou appelée également chromatographie d'exclusion stérique (SEC, size exclusion chromatographie). Des conditions opératoires utilisables pour mettre en oeuvre cette méthode sont les suivantes : La préparation des échantillons est faite à une concentration de 1g/l, avec une durée de dissolution de 4heures minimum. Les conditions de mesure sont les suivantes : solvant : THF ; température : 30°C, volume injecté : 100µl, détection : indice de réfraction; étalonnage avec des standards Polystyrène (PS) en utilisant la calibration universelle. Les masses sont exprimées en équivalant PMMA grâce aux coefficients de Mark-Houwink-Sakurada. Pour cette détermination, on peut utiliser par exemple un appareillage de la société WATERS (Milford USA).

Des essais de résistance chimique (selon la méthode EN 13559) ont en effet montré de façon surprenante que les PMMA de masse moléculaire inférieure à 90000 g/mol ne permettent pas de résister aux produits utilisés pour le nettoyage des photobioréacteurs. Par ailleurs, les PMMA de masse moléculaire inférieure à 90000 g/mol s'avèrent fragiles en enceinte humide et chaude (85% d'humidité et température 85°C), et ne sont donc pas adaptés à l'utilisation envisagée.

Les polymères méthacryliques commercialisés sous la marque Altuglas^{®}, en particulier le grade HCR-3, sont parfaitement adaptés pour l'invention,

La composition à base d'au moins un polymère méthacrylique selon l'invention peut être renforcée à l'impact à l'aide d'au moins un modifiant choc. On utilise avantageusement une extrudeuse pour réaliser le mélange. Le modifiant choc peut être par exemple un élastomère acrylique. L'élastomère acrylique peut être un copolymère séquencé présentant au moins une séquence élastomérique. Par exemple, il peut s'agir d'un copolymère styrène-butadiène-méthacrylate de méthyle ou méthacrylate de méthyle-acrylate de butyle-méthacrylate de méthyle. Le modifiant choc peut se présenter aussi sous forme de fines particules multicouches, appelées core-shell (noyau-écorce), ayant au moins une couche élastomérique (ou molle), c'est-à-dire une couche formée d'un polymère ayant une T_{g} inférieure à -5°C et au moins une couche rigide (ou dure), c'est-à-dire formée d'un polymère ayant une T_{g} supérieure à 25°C.

La composition selon l'invention peut comprendre en outre des additifs classiquement employés choisis parmi les stabilisants thermiques, par exemple le terdocécyldisulfure (DtDDS) ou l'Irganox^{©} 1076 ; les lubrifiants, par exemple l'acide stéarique ou l'alcool stéarylique ; les ignifugeants, par exemple le trioxyde d'antimoine ou un phosphate ester bromé ou chloré ; les pigments organiques ou inorganiques ; les anti-UV, par exemple le Tinuvin^{©} P ; les anti-oxydants, tels que des composés phénoliques encombrés ; les antistatiques.

La composition selon l'invention comprend au moins un additif antifouling comme defini par les revendications afin d'éviter les phénomènes d'accrochage et de salissures en présence du milieu de culture et/ou en présence d'air. L'utilisation de tels additifs à la composition méthacrylique permet en outre de mettre en oeuvre des milieux de cultures concentrés sans voir apparaitre les problèmes d'accrochage des organismes photosensibles sur les parois en contact avec le milieu organique et/ou en contact avec l'air.

Comme additive antifouling et/ou antistatique optionale, on peut utiliser notamment un copolymère à blocs polyamide et blocs polyéther (appelé aussi PEBA selon l'IUPAC),

Les PEBA ou Polyether-Bloc-Amide, tels que ceux commercialisés par la Société Arkema sous le nom Pebax^{®}, résultent de la polycondensation de blocs polyamides PA à extrémités réactives avec des blocs polyéthers PE à extrémités réactives, telles que, entre autres :
1) blocs polyamides à bouts de chaîne diamines avec des blocs polyoxyalkylènes à bouts de chaînes dicarboxyliques.
2) blocs polyamides à bouts de chaînes dicarboxyliques avec des blocs polyoxyalkylènes à bouts de chaînes diamines, obtenues par cyanoéthylation et hydrogénation de blocs polyoxyalkylène alpha-oméga dihydroxylées aliphatiques appelées polyétherdiols
3) blocs polyamides à bouts de chaînes dicarboxyliques avec des polyétherdiols, les produits obtenus étant, dans ce cas particulier, des polyétheresteramides.

Les blocs polyamides à bouts de chaînes dicarboxyliques proviennent, par exemple, de la condensation de précurseurs de polyamides en présence d'un diacide carboxylique limiteur de chaîne.

Les blocs polyamides à bouts de chaînes diamines proviennent par exemple de la condensation de précurseurs de polyamides en présence d'une diamine limiteur de chaîne.

La masse molaire en nombre Mn des blocs polyamides est comprise dans la gamme allant de 400 à 20000 g/mol, de préférence de 500 à 10000 g/mol, et de préférence encore de 600 et 6000 g/mol.

Les polymères à blocs polyamides et blocs polyéthers peuvent aussi comprendre des motifs répartis de façon aléatoire.

Les blocs polyamides peuvent comporter des homopolyamides ou des copolyamides.

Trois types de polyamides peuvent entrer dans la composition de ces blocs PA.

Selon un premier type, les blocs polyamide proviennent de la condensation d'au moins un diacide carboxylique (aliphatique, cycloaliphatique ou aromatique) en particulier ceux ayant de 4 à 36 atomes de carbone, de préférence ceux ayant de 6 à 18 atomes de carbone et d'au moins une diamine (aliphatique, cycloaliphatique ou aromatique) choisie en particulier parmi celles ayant de 2 à 20 atomes de carbone, de préférence celles ayant de 6 à 15 atomes de carbone.

A titre d'exemples de diacides aliphatiques, on peut citer les acides butanedioïque, adipique, subérique, azélaïque, sébacique, dodécanedicarboxylique, myristique, tétradécanedicarboxylique, hexadécanedicarboxylique, octadécanedicarboxylique et les acides gras dimérisés,

A titre d'exemples de diacides cycloaliphatiques, on peut citer l'acide 1,4-cyclohexyldicarboxylique.

A titre d'exemples de diacides aromatiques, on peut citer les acides téréphtalique (T) et isophtalique (I).

A titre d'exemples de diamines aliphatiques, on peut citer la tétraméthylène diamine, l'hexaméthylènediamine, la 1,10-décaméthylènediamine, la dodécaméthylènediamine, la triméthylhexaméthylène diamine.

A titre d'exemple de diamines cycloaliphatiques, on peut citer les isomères des bis-(4-aminocyclohexyl)-méthane (BACM ou PACM), bis-(3-méthyl-4-aminocyclohexyl)méthane (BMACM ou MACM), et 2-2-bis-(3-méthyl-4-aminocyclohexyl)-propane (BMACP), l'isophoronediamine (IPDA), la 2,6-bis-(aminométhyl)-norbornane (BAMN) et la pipérazine (Pip).

Avantageusement, le copolymère comprend au moins un bloc PA à base de PA 4.4, PA 4.6, PA 4.9, PA 4.10, PA 4.12, PA 4.14, PA 4.16, PA 4.18, PA 4.36, PA 6.4, PA 6.6, PA 6.9, PA 6.10, PA 6.12, PA 6.13, PA 6.14, PA 6.16, PA 6.18, PA 6.36, PA 9.4, PA 9.6, PA 9.10, PA 9.12, PA 9.14, PA 9.18, PA 9.36, PA 10.4, PA 10.6, PA 10.9, PA 10.10, PA 10.12, PA 10.13, PA 10.14, PA 10.16, PA 10.18, PA 10.36, PA 10.T, PA BMACM.4, PA BMACM.6, PA BMACM.9, PA BMACM.10, PA BMACM.12, PA BMACM.14, PA BMACM.16, PA BMACM.18, PA BMACM.36, PA PACM.4, PA PACM.6, PA PACM.9, PA PACM.10, PA PACM.12, PA PACM.14, PA PACM.16, PA PACM.18, PA PACM.36, PA Pip.4, PA Pip.6, PA Pip.9, PA Pip.10, PA Pip.12, PA Pip.14, PA Pip.16, PA Pip.18 et/ou PA Pip.36, et leurs mélanges.

Selon un deuxième type, les blocs polyamides résultent de la condensation d'un ou plusieurs acides alpha oméga aminocarboxyliques et/ou d'un ou plusieurs lactames ayant de 6 à 12 atomes de carbone en présence d'un diacide carboxylique ayant de 4 à 12 atomes de carbone ou d'une diamine.

A titre d'exemples de lactames, on peut citer le caprolactame, l'oenantholactame et le lauryllactame.

A titre d'exemples d'acide alpha omega amino carboxylique, on peut citer les acides aminocaproïque, amino-7-heptanoïque, amino-11-undécanoïque et amino-12-dodécanoïque.

Les diacides carboxyliques ou les diamines peuvent être choisis parmi ceux cités plus haut.

Avantageusement les blocs polyamides du deuxième type sont en polyamide 11, en polyamide 12 ou en polyamide 6.

Selon un troisième type, les blocs polyamides résultent de la condensation d'au moins un monomère du premier type avec au moins un monomère du deuxième type. En d'autres termes, les blocs polyamides résultent de la condensation d'au moins un acide alpha oméga aminocarboxylique (ou un lactame), avec au moins une diamine et un diacide carboxylique.

Dans ce cas, on prépare les blocs PA par polycondensation :
- de la ou des diamines aliphatiques, cycloaliphatiques ou aromatiques ayant X atomes de carbone ;
- du ou des diacides carboxyliques ayant Y atomes de carbone ; et
- du ou des comonomères {Z}, choisis parmi les lactames et les acides alpha-oméga aminocarboxyliques ayant Z atomes de carbone ;
- en présence d'un limiteur de chaîne choisi parmi les diacides carboxyliques ou diamines ou d'un excès de diacide ou de diamine utilisé comme unité structurale.

Avantageusement, on utilise comme limiteur de chaîne le diacide carboxylique ayant Y atomes de carbone, que l'on introduit en excès par rapport à la stoechiométrie de la ou des diamines.

Selon une autre variante (cas des copolymères, c'est-à-dire des copolyamides), les blocs polyamides résultent de la condensation d'au moins deux acides alpha oméga aminocarboxyliques différents ou d'au moins deux lactames différents ayant de 6 à 12 atomes de carbone ou d'un lactame et d'un acide aminocarboxylique n'ayant pas le même nombre d'atomes de carbone en présence éventuelle d'un limiteur de chaîne.

A titre d'exemples de blocs polyamides du troisième type, on peut citer ceux formés par les polyamides (copolyamides) suivants :
- PA 6/6.6 dans lequel 6 désigne du caprolactame et 6.6 désigne un monomère résultant de la condensation de l'hexaméthylènediamine avec de l'acide adipique.
- PA 6.6/Pip.10/12 dans laquelle 6.6 désigne un monomère résultant de la condensation de l'hexaméthylènediamine avec de l'acide adipique. Pip.10 désigne un monomère résultant de la condensation de la pipérazine avec l'acide sébacique. 12 désigne du Sauryllactame.
- PA 6.6/6.10/11/12 dans laquelle 6.6 désigne monomère résultant de la condensation de l'hexaméthylènediamine avec de l'acide adipique. 6.10 désigne un monomère résultant de la condensation de l'hexaméthylènediamine avec l'acide sébacique. 11 désigne l'acide amino-11-undécanoïque. 12 désigne du lauryllactame.

A titre d'exemples, on peut encore citer le PA 10.10/11, PA 6.10/11, PA10.12/11, PA 10.10/11/12, PA 6.10/10.10/11, PA 6.10/6.12/11, PA 6.10/6.12/10.10.

Dans les PEBA, les blocs polyéthers peuvent représenter 1 à 99%, et de préférence 5 à 90 % en poids du copolymère à blocs polyamides et polyéthers, encore plus préférentiellement de 10 à 50% en poids. La masse molaire Mn des blocs polyéther est comprise dans la gamme allant de 100 à 6 000 g/mol et de préférence de 200 à 3 000 g/mol, encore plus préférentiellement de 250 à 2000 g/mol.

Par blocs polyéther (abrégé ci-après PE) on entend les polyalkylènes éthers polyols, notamment des polyalkylènes éther diols, tels que le poly(éthylène glycol) (PEG), le poly{1,2-propylène glycol) (PPG), le polytétraméthylène éther glycol (PTMG), le polyhexaméthylène glycol, le poly(1,3-propylène glycol) (PO3G), les poly(3-alkyl tétrahydrofurane) en particulier le poly(3-méthyltétrahydrofurane (poly(3MeTHF)), et leurs mélanges. On peut également envisager un bloc PE de type «copolyéther» à blocs ou statistique contenant un enchaînement d'au moins deux types de PE cités ci-dessus. Les blocs polyéther peuvent également comprendre des blocs obtenus par oxyéthylation de bisphenols, tels que par exemple le bisphenol A. Ces derniers produits sont décrits dans le brevet EP 613 919.

Les blocs polyéthers peuvent aussi comprendre des amines primaires éthoxylées. On utilise avantageusement aussi ces blocs. A titre d'exemple d'amines primaires éthoxylées on peut citer les produits de formule : 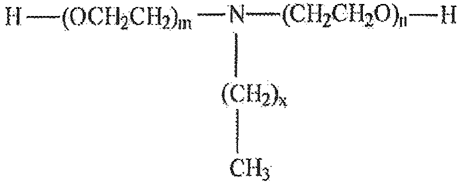 dans laquelle m et n sont compris entre 1 et 20 et x entre 8 et 18. Ces produits sont disponibles dans le commerce sous la marque NORAMOX^{®} de la société CECA et sous la marque GENAMIN^{®} de la société CLARIANT.

Ainsi, les fins de chaînes des blocs PE peuvent être diOH, diNH₂, diisocyanate ou diacide suivant leur procédé de synthèse.

Les blocs PE à bouts de chaîne NH₂, peuvent être obtenus par cyanoacétylation de séquences polyoxyalkylène alpha-oméga dihydroxylées aliphatiques appelées polétherdiols telles que les Jeffamines^{®} D300, D400, D2000, ED-600, ED-900, ED2003, les Elastamines^{®} RP-409, RP-2009, RT-1000, RE-600, RE-900, RE-2000, HT-1700, HE-180 de la société Huntsman. De tels blocs sont décrits dans les brevets JP 2004346274, JP 2004352794 et EP1482011.

La préparation des copolymères à bloc(s) polyamide et bloc(s) polyéther PEBA comprend tout moyen permettant d'accrocher les blocs polyamide (bloc PA) et les blocs polyéther (bloc PE) selon la présente invention. En pratique, on utilise essentiellement deux procédés l'un dit en 2 étapes, l'autre en une étape.

La méthode générale de préparation en deux étapes des copolymères PEBA ayant des liaisons ester entre les blocs PA et les blocs PE est connue et est décrite, par exemple, dans le brevet français FR 2 846 332. La méthode générale de préparation des copolymères PEBA ayant des liaisons amide entre les blocs PA et les blocs PE est connue et décrite, par exemple dans le brevet européen EP 1 482 011.

Avantageusement, on utilise les méthodes en deux étapes ou en une seule étape décrites dans le document WO 01/18111.

Selon l'invention, on utilise en outre avantageusement comme additifs antifouling et/ou antistatiques optionnels:
- les copolymères à blocs polyamides et blocs polyéthers comprenant essentiellement des motifs oxyde d'éthylène et dans lequels les blocs polyamides sont des copolyamides résultant de la condensation d'au moins un acide alpha oméga aminocarboxylique (ou un lactame), au moins une diamine et au moins un acide dicarboxylique, comme décrits dans le document EP 1 046 675,
- les poylétheresteramides ayant des blocs polyamide comprenant des sulfonates d'acides dicarboxyliques soit comme limiteurs de chaîne du bloc polyamide, soit associés à une diamine comme l'un des monomères constitutifs du bloc polyamide et ayant des blocs polyéthers consitutés essentiellement de motifs oxyde d'alkylène, comme décrits dans le document WO 01/29113.
- Un mélange d'un copolymère à blocs polyamides et blocs polyéthers et d'un polymère ou oligomère comprenant dans sa chaine au moins une fonction ionique et choisi parmi les polyamides, les copolymères à blocs polyamides et blocs polyéthers, les polyesters ou polyétheramides thermoplastiques, les copolymères à blocs polyesters et blocs polyéthers, les polyéthers et les polyuréthanes, comme décrits dans le document EP 1 262 527.

Selon un mode de réalisation préféré de l'invention, on utilise comme additifs antifouling et/ou antistatique les copolymères ayant des blocs polyamide 12 et des blocs polyéther comprenant essentiellement des motifs oxyde d'éthylène. En particulier, on peut utiliser le Pebax^{®}MV 1074 ayant des blocs polyamide 12 de masse molaire moyenne en nombre 1500 et des blocs PEG de masse molaire moyenne en nombre 1500.

Les additifs antifouling et/ou antistatique décrits précédemment sont utilisés de préférence à une teneur pouvant aller de 3 à 15 % en poids par rapport à la composition totale, c'est-à-dire permettant au matériau d'avoir un effet glissant et de ne pas attirer les poussières. Selon la teneur en additif, on pourra obtenir un polymère méthacrylique présentant des propriétés antifouling ou un polymère méthacrylique présentant des propriétés antistatiques. A titre d'exemple, une composition de PMMA comprenant de 5% à 10%, plus particulièrement de 6% à 8% d'un PEBA, conduit à une composition présentant de bonnes propriétés antistatiques. De préférence, pour obtenir un polymère méthacrylique présentant des propriétés antifouling, l'additif sera présent à une teneur allant de 3% à 7% en poids par rapport à la composition totale.

La propriété antistatique et/ou antifouling d'un polymère peut être mesurée par sa résistivité surfacique. A titre d'exemple, un film de 500 µm de PMMA seul de masse moléculaire égale à 150000g/mol, présente une résistivité surfacique de 126 10⁺¹⁵ Ohm. L'ajout de 7% en poids et 10% en poids du PEBAX^{®} MV1074 dans le PMMA permet respectivement d'obtenir des résistivités surfaciques mesurées sur film de 500µm de 136 10⁺¹² et 47 10⁺¹² Ohm, les mesures de résistivité surfacique ayant été réalisées à l'aide d'une cellule Keithley reliée à un électromètre, selon la norme ASTM D257, avec une tension de mesure de 500 Volts.

La propriété antifouling (effet glissant) peut être quantifiée par des mesures de coefficient de friction selon la norme ASTM D1894-08 qui permet de déterminer la force nécessaire pour faire glisser un pavé sur la surface de la pièce à évaluer. Les essais réalisés suivant cette norme donnent des coefficients de friction statique et dynamique pour un Altuglas standard de type V046 de 0,154 et 0,107. Ce grade additivé avec 5% de Pebax MV1074 a des valeurs de coefficients de friction statique et dynamique de 0,074 et 0,053.

Selon l'invention, on utilise comme additif antifouling un polymère fluoré permettant d'apporter des propriétés antifouling, mais aussi une bonne résistance, notamment aux UV et aux produits chimiques.

Le polymère fluoré est choisi parmi :
- les homo- et copolymères du fluorure de vinylidène (VDF, CH₂=CF₂) contenant au moins 50% en poids de VDF. Le comonomère du VDF peut être choisi parmi le chlorotrifluoroéthylène (CTFE), l'hexafluoropropylène (HFP), le trifluoroéthylène (VF₃) et le tétrafluoroéthylène (TFE) ;

Le polymère fluoré est un PVDF homo- ou copolymère. Ce polymère fluoré présente en effet une bonne résistance chimique, notamment aux UV et aux produits chimiques, et il est complètement miscible dans une matrice de polymère méthacrylique. De préférence le PVDF contient, en poids, au moins 50% de VDF, plus préférentiellement au moins 75% et mieux encore au moins 85%. Le comonomère est avantageusement l'HFP.

Ainsi, les PVDF commercialisés sous la marque KYNAR^{®}, en particulier les grades 710, 720 ou 740 sont parfaitement adaptés pour cette formulation.

Le polymère fluoré est présent de préférence à une teneur pouvant aller de 5 à 60 % en poids par rapport à la composition totale de préférence de 20 à 40 %.

La composition selon l'invention peut se préparer par les techniques habituelles des thermoplastiques telles que par exemple par extrusion ou à l'aide de mélangeurs bivis ou encore à l'aide d'un appareil du type Ko-malaxeur Buss^{®}.

La composition selon l'invention peut être mise sous la forme de plaques, profilés creux en forme de « H » ou de « I » par exemple, ou de cylindres tels que des tubes selon les procédés classiques d'extrusion ou d'injection.

Ainsi, l'invention est relative à une structure multicouche transparente comprenant au moins une couche d'un polymère méthacrylique et une couche comprenant au moins un additif antifouling et/ou antistatique, ainsi qu'à son utilisation pour la construction d'installations destinées à la culture d'organismes photosensibles, tels que microorganismes, microalgues, bactéries photosynthétiques, planctons tel que défini par les revendications.

La structure multicouche comprend au moins :
- une couche d'au moins un polymère méthacrylique tel que défini précédemment
- une couche comprenant au moins un additif antifouling susceptible d'être en contact avec un milieu de culture, les couches étant disposées l'une sur l'autre.

La couche en contact avec le milieu de culture est constituée, d'un polymère méthacrylique antifouling défini dans la présente invention par un polymère méthacrylique tel que défini précédemment, additivé d'au moins un additif antifouling choisi parmi des PVDF homo- et copolymères, tel que défini par des revendications.

De manière préférée, la structure multicouche comprend dans l'ordre au moins :
- une couche comprenant au moins un additif antistatique susceptible d'être en contact avec l'air ou le milieu extérieur
- une couche d'au moins un polymère méthacrylique tel que défini précédemment
- une couche comprenant au moins un additif antifouling susceptible d'être en contact avec un milieu de culture.

Avantageusement, la couche en contact avec l'air ou le milieu extérieur est constituée d'un polymère méthacrylique antistatique, défini dans la présente invention par un polymère méthacrylique additivé de 5 à 10% d'un additif antistatique choisi parmi les copolymères à blocs polyamides et blocs polyéthers.

La structure mono ou multicouche présente un épaisseur comprise entre 200 µm et 12 mm, de manière préférée entre 500 µm et 7 mm.

La couche antistatique ou antifouling présente une épaisseur comprise entre 50 µm et 2 mm, et de préférence entre 100 µm et 1 mm.

Les structures multicouches peuvent être coextrudées, comprimées à chaud, coextrudées-laminées, et de préférence sont coextrudées, permettant d'obtenir des tubes ou des plaques multicouches.

L'invention est relative aussi à un tube multicouche pouvant présenter un diamètre de 2 à 100 cm, de préférence de 10 à 60 cm, et une longueur de 1 à 50 mètres.

## Revendications

1. Structure multicouche pour la construction d'installations destinées à la culture d'organismes photosensibles, la structure comprenant au moins :
- une couche d'au moins un polymère méthacrylique
- une couche comprenant au moins un additif antifouling susceptible d'être en contact avec un milieu de culture, les couches étant disposées l'une sur l'autre,
**caractérisée en ce que** la couche susceptible d'être en contact avec le milieu de culture est constituée d'un polymère méthacrylique antifouling défini par un polymère méthacrylique additivé d'au moins un additif antifouling choisi parmides PVDF homo - et copolymères , et **caractérisé en ce que** le polymère méthacrylique est un PMMA - homopolymère du méthacrylate de méthyle MAM ou un copolymère du méthacrylate de méthyle (MAM), comprenant en poids au moins 50% de MAM - de masse moléculaire en poids allant de 90000 g/mol à 170000 g/mol (étalon PMMA);
**caractérisée en ce que** la structure multicouche est sous forme de tube, profilé ou de plaque.

2. Structure multicouche selon la revendication 1 comprenant dans l'ordre au moins :
- une couche comprenant au moins un additif antistatique susceptible d'être en contact avec l'air ou le milieu extérieur
- une couche d'au moins un polymère méthacrylique
- une couche comprenant au moins un additif antifouling susceptible d'être en contact avec un milieu de culture.

3. Structure selon la revendication 2 **caractérisée en ce que** la couche en contact avec l'air ou le milieu extérieur est constituée d'un polymère méthacrylique antistatique, défini par un polymère méthacrylique additivé de 5 à 10% d'un additif antistatique choisi parmi les copolymères à blocs polyamides et blocs polyéthers.

4. Tube multicouche selon la revendication 1, **caractérisé en ce qu'**il présente un diamètre allant de 2 à 100 cm et une longueur de 1 à 50 m.

5. Plaque multicouche selon la revendication 1, **caractérisée en ce qu'**elle présente une épaisseur allant de 1 à 100 mm.

6. Utilisation d'une tube multicouche selon l'une des revendications 1 à 4, ou d'une plaque selon la revendication 5 pour la construction d'installations destinées à la culture d'organismes photosensibles, tels que microorganismes, microalgues, bactéries photosynthétiques, planctons.

## Patentansprüche

1. Mehrschichtige Struktur für den Bau von Vorrichtungen zur Kultivierung von photosensitiven Organismen, die Struktur bestehend mindestens aus:
- einer Schicht mit mindestens einem Methacrylpolymer,
- einer Schicht mit mindestens einem Antifouling-Additiv, das Kontakt zu einem Kulturmedium haben kann, wobei diese Schichten übereinander aufgebracht sind,
**dadurch gekennzeichnet, dass** die Schicht, die Kontakt mit dem Kulturmedium haben kann, aus einem Antifouling-Methacrylpolymer besteht, das durch ein Methacrylpolymer definiert ist, dem mindestens ein Antifouling-Additiv zugesetzt ist, das aus den PVDF-Homo- und Copolymeren ausgewählt ist und **gekennzeichnet ist dadurch**, dass das Methacrylpolymer ein PMMA-Homopolymer aus Methylmethacrylat (MMA) oder ein Copolymer aus Methylmethacrylat (MMA) mit mindestens 50 Gew.-% MMA - mit einer gewichtsbezogenen mittleren molekularen Masse von 90.000 g/mol bis 170.000 g/mol (PMMA-Standard) ist,
**dadurch** gekennzeichnet, dass die mehrschichtige Struktur in Form von Rohren, Profilen oder Platten vorliegt.

2. Mehrschichtige Struktur nach Anspruch 1, die in der angegebenen Reihenfolge mindestens folgendes umfasst:
- eine Schicht mit mindestens einem Antistatik-Additiv, das Kontakt zu Luft oder einem Außenmedium haben kann,
- eine Schicht mit mindestens einem Methacrylpolymer,
- eine Schicht mit mindestens einem Antifouling-Additiv, das Kontakt zu einem Kulturmedium haben kann.

3. Struktur nach Anspruch 2, **dadurch gekennzeichnet, dass** die Schicht in Kontakt mit der Luft oder dem Außenmedium aus einem antistatischen Methacrylpolymer besteht, das durch ein Methacrylpolymer definiert ist, dem 5 bis 10 % eines Antistatik-Additivs zugesetzt sind, das aus den Polyamidblock- und Polyetherblock-Copolymeren ausgewählt ist.

4. Mehrschichtiges Rohr nach Anspruch 1, **dadurch gekennzeichnet, dass** dieses Rohr einen Durchmesser von 2 bis 100 cm und eine Länge von 1 bis 50 m aufweist.

5. Mehrschichtige Platte nach Anspruch 1, **dadurch gekennzeichnet, dass** diese Platte eine Dicke von 1 bis 100 mm aufweist.

6. Verwendung eines mehrschichtigen Rohrs nach einem der Ansprüche 1 bis 4 oder einer Platte nach Anspruch 5 für den Bau von Vorrichtungen zur Kultivierung von photosensitiven Organismen, z. B. Mikroorganismen, Mikroalgen, photosynthetischen Bakterien, Plankton.

## Claims

1. A multilayer structure for the construction of systems used to culture photosensitive organisms, the structure comprising at least:
- a layer of at least one methacrylic polymer;
- a layer comprising at least one antifouling additive likely to be in contact with a culture medium, the layers being placed one on top of the other;
**characterised in that** the layer likely to be in contact with the culture medium consists of an antifouling methacrylic polymer defined by a methacrylic polymer additivated with at least one antifouling additive chosen among PVDF homo- and copolymers, and **characterised in that** the methacrylic polymer is a PMMA - homopolymer of methyl methacrylate (MMA) or a copolymer of methyl methacrylate (MMA), comprising at least 50% by weight of MMA - having a weight-average molecular mass ranging from 90,000 g/mol to 170,000 g/mol (PMMA standard);
**characterised in that** the multilayer structure is in the form of a tube, profile or plate.

2. A multilayer structure according to claim 1, comprising, in order, at least:
- a layer comprising at least one antistatic additive likely to come into contact with air or the external environment;
- a layer of at least one methacrylic polymer;
- a layer comprising at least one antifouling additive likely to be in contact with a culture medium.

3. A structure according to claim 2, **characterised in that** the layer in contact with air or with the external environment consists of an antistatic methacrylic polymer, defined by a methacrylic polymer additivated with from 5% to 10% of an antistatic additive chosen among the polyamide-polyether block copolymers.

4. A multilayer tube according to claim 1, **characterised in that** it has a diameter ranging from 2 to 100 cm and a length of from 1 to 50 m.

5. A multilayer plate according to claim 1, **characterised in that** it has a thickness ranging from 1 to 100 mm.

6. Use of a multilayer tube according to one of claims 1 to 4, or of a plate according to claim 5, for the construction of systems used to culture photosensitive organisms such as microorganisms, micro-algae, photosynthetic bacteria, plankton.
